# EUROPEAN PATENT SPECIFICATION

(11) **EP 2 675 427 B1**
(45) Date of publication and mention of the grant of the patent: **05.08.2015**
(21) Application number: 12700699.7
(22) Date of filing: 20.01.2012
(51) Int. Cl.: A61K 8/06, A61K 8/365, A61K 8/60, A61K 8/90, A61Q 19/00, A61K 8/892, A61K 8/893, A61K 8/86

(54) **LEAVE-ON NON-SOLID SKIN CONDITIONING COMPOSITION WHICH HAS A CONTINUOUS PHASE AND CONTAINS 12 - HYDROXYSTEARIC ACID**
NICHT FESTE HAUTKONDITIONIERUNGSZUSAMMENSETZUNG MIT EINER KONTINUIERLICHEN PHASE UND 12-HYDROXYSTEARINSÄURE
COMPOSITION SANS-RINÇAGE NON-SOLIDE CONDITIONNANTE POUR LA PEAU À PHASE CONTINUE HUILEUSE CONTENANT DE L'ACIDE 12-HYDROXYSTÉARIQUE

(30) Priority: 17.02.2011 US 201113029562
(43) Date of publication of application: 25.12.2013
(73) Proprietor: Unilever PLC, London, Greater London EC4Y 0DY (GB); Unilever N.V., 3013 AL Rotterdam (NL)
(72) Inventor: PEHRATOVIC, Hasiba, Trumbull, Connecticut 06611 (US); MOADDEL, Teanoosh, Trumbull, Connecticut 06611 (US); DOBKOWSKI, Brian, John, Trumbull, Connecticut 06611 (US)
(74) Representative: Corsten, Michael Allan
(86) International application number: PCT/EP2012/050851
(87) International publication number: WO 2012/110276

(56) References cited:
- EP-A1- 1 623 696
- EP-A1- 1 964 546
- US-A1- 2003 113 427
- US-A1- 2009 130 029
- US-A1- 2009 317 341

## Description

### BACKGROUND OF THE INVENTION

Salts of 12-hydroxystearic acid, i.e. soaps, have been described in wash-off body cleansing compositions (JP 4 266 904; JP 59/227,999, JP 56/074,197). 12-hydroxystearic acid (hereinafter "12HSA") is reported to have a wide variety of beneficial cosmetic effects on skin, e.g. it is a known PPAR-alpha (peroxisome proliferator activated receptors sub-type alpha) activator, skin lightening agent, and a sebum secretion inhibitor. See e.g. Alaluf et al. US 6 423 325, Mayes et al. US 6 713 051, WO 2006/056283 (Hindustan Lever), Madison US 2009/0317341, Minami et al. US 6 197 343, Granger et al. US 2004/0043044. As such, cosmetic leave-on products containing 12HSA are highly desirable. JP 09-048962 describes the use of 12HSA or its salt as an effective constituent of a solidification inhibitor, to inhibit solidification of a liquid detergent or a liquid cosmetic; all the examples containing a fully neutralized salt of 12HSA. Unfortunately, 12HSA is a solid and has no water solubility and limited oil solubility. Indeed, 12HSA has traditionally been used as gelling agent e.g. in lipsticks and anti-perspirant compositions. See also EP 0 129 528, US 6 680 285, Abbas et al. US 6 680 285, Tanner et al. US 5 759 524, WO 95/31961 (Procter & Gamble), Kawa et al., US 2004/0044078 (describing the use of 12HSA to increase viscosity of cosmetic compositions), and JP 2010/138,110. Salts of 12HSA are only marginally more water-soluble. Thus, non-solid skin conditioning leave-on compositions containing 12HSA are highly desirable, and specifically oil-continuous compositions.

### SUMMARY OF THE INVENTION

According to the present invention, structurally reversible non-solid oil-continuous skin conditioning compositions have been prepared that contain 12HSA. Structural reversibility of 12HSA containing formulations is crucial, to prevent solidifying of the product on storage, or upon spreading on the skin, rendering it unusable and/or not bioavailable. The invention also includes methods of making and using the compositions.

### DETAILED DESCRIPTION OF THE INVENTION

Except in the examples, or where otherwise explicitly indicated, all numbers in this description indicating amounts of material or conditions of reaction, physical properties of materials and/or use are to be understood as modified by the word "about." All amounts are by weight of the final composition, unless otherwise specified.

It should be noted that in specifying any range of concentration or amount, any particular upper concentration can be associated with any particular lower concentration or amount.

For the avoidance of doubt, the word "comprising" is intended to mean "including" but not necessarily "consisting of" or "composed of." In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

"Conditioning" as used herein means prevention and treatment of dry skin, acne, photo-damaged skin, appearance of wrinkles, age spots, aged skin, increasing stratum corneum flexibility, lightening skin color, controlling sebum excretion and generally increasing the quality of skin. The composition may be used to improve skin desquamation and epidermal differentiation and improve skin appearance or general aesthetics.

"Leave-on" as used herein means compositions that are applied to the skin and are not intended to be washed or rinsed off for some period of time, as contrasted with cleansing or wash-off or rinse-off compositions.

"Non-solid" as used herein means that the composition has a measurable viscosity (measurable for instance with a Brookfield Viscometer DV-I + (20RPM, RV6, 30 Seconds) in the range of from 1 Pas to 500 Pas, preferably from 2 Pas to 100 Pas, more preferably from 3 Pas to 50 Pas at room temperature.

"Stable" or "Stability" as used herein means that composition does not separate and is not grainy and does not curdle and does not solidify or form a stiff gel.

"Structurally reversible" as used herein means that compositions (i) do not form a stiff gel or solid, do not separate, and do not curdle when initially formulated; and (ii) maintain structural integrity (i.e., the initial appearance and structure) after having been exposed to 50°C for 24 hours, and then allowed to cool to room temperature. Structural reversibility is gauged by spreading 3-5 grams of a formulation on a dark smooth flat surface (e.g. black lab bench top) and visually observing the film texture. Samples that display structural reversibility have a smooth homogeneous appearance whereas samples that do not display structural reversibility have a grainy surface appearance.

### 12HSA

The inventive compositions include 12HSA. The compositions typically contain at least 40 % of the total 12HSA in its acid form, preferably at least 50 %, more preferably at least 60 % in order to optimize bioavailability, and therefore efficacy. As with other fatty acids the apparent pKa for 12HSA is expected to be greater than 8. At the pKa, the fatty acid will exist as 50 % soap and 50 % acid. Therefore, preferably the pH of the inventive compositions is less than about 8, more preferably is in the range of from 3.5 to 8.0, most preferably is from 5 to 7.8. 12HSA is included in the inventive compositions in an amount of from 0.01 to 15 %, preferably from 0. 1 to 12 %, more preferably from 0.5 to 10 %, and optimally from 1 to 5 %. The amounts of 12HSA herein include both acid and salt amounts. The amounts of 12HSA or salts thereof are not meant to be included within the surfactants amounts herein.

### LOW HLB NONIONIC SUGAR SURFACTANT

Non-ionic sugar surfactants suitable for inclusion into inventive composition are sugar surfactants with an HLB below 9, preferably HLB below 7, and are selected from the group consisting of sugar fatty acid esters. It has been found, as part of the present invention, that nonionic sugar surfactants with HLB above 9 do not attain structurally reversible oil-continuous compositions. The nonionic surfactants described below are suitable for use in the present invention, as long as they are selected also to have a low HLB.

### Sugar fatty acid esters

Esters of fatty acid and sugar include esters or mixtures of esters of linear or branched and saturated or unsaturated C₁₂ to C₂₂ fatty acids and of sucrose, maltose, glucose, fructose, mannose, galactose, arabinose, xylose, lactose, trehalose or methylglucose. These esters are preferably chosen from mono-, di-, tri- and tetraesters, polyesters and their mixtures. The C₁₂ to C₂₂ fatty acids includes C₁₂, C₁₃, C₁₄, C₁₅, C₁₆, C₁₇, C₁₈, C₁₉, C₂₀, C₂₁, and C₂₂ in any subrange or combination. These esters are preferably chosen from stearates, behenates, cocoates, arachidonates, palmitates, myristates, laurates, carprates, oleates, laurates and their mixtures. These compounds can be used in particular as emulsifying surfactants. Mixtures of these derivatives are possible.

Sucrose esters are preferably used. Preferable sucrose esters include sucrose cocoate, sucrose monooctanoate, sucrose monodecanoate, sucrose mono- or dilaurate, sucrose monomyristate, sucrose mono- or dipalmitate, sucrose mono- and distearate, sucrose mono-, di- or trioleate, sucrose mono- or dilinoleate, sucrose polyesters, such as sucrose pentaoleate, hexaoleate, heptaoleate or octooleate, and mixed esters, such as sucrose palmitate/stearate. Mixtures are possible.

Preferable esters or mixtures of esters of fatty acid and of sucrose include those sold by the company Crodesta under the names Crodesta F10, respectively denoting sucrose distearate. Use may also be made of those sold by the company MMP Inc. under the name Sisterna SP10-C corresponding to sucrose polystearate. Mention may also be made of the sucrose mono- and dipalmitate/stearate sold by the company Goldschmidt under the name "Tegosoft PSE". Use may also be made of a mixture of these various products.

The sugar ester can also be present in admixture with another compound not derived from sugar; and a preferred example includes the mixture of sorbitan stearate and of sucrose cocoate sold under the name "Arlatone 2121" by the company ICI. Other preferable sugar esters include, for example, glucose trioleate, galactose di-, tri-, tetra- or pentaoleate, arabinose di-, tri- or tetralinoleate or xylose di-, tri- or tetralinoleate. Mixtures are possible.

Other preferable esters or mixtures of esters of fatty acid and of methylglucose include the distearate of methylglucose and of polyglycerol-3 sold by the company Goldschmidt under the name of Tegocare 450. Still other preferable esters include the oleate, isostearate, or sesquistearate of methyl glucose. These are sold under the tradename Glucate DO, Isolan IS, glucate SS. Glucate DO and SS are available from Lubrizol/Neveone and Isolan IS from Evonik Goldschmidt GmBH. Mention may also be made of glucose or maltose monoesters, such as methyl O-hexadecanoyl-6-D-glucoside and (lacuna) O-hexadecanoyl-6-D-maltose.

Other sugar fatty acid ester derivatives which can be used in the composition of the invention include sugar fatty esters which are optionally oxyalkylenated (oxyethylenated and/or oxypropylenated) or polyglycerolated. Preferable oxyethylenated esters of fatty acid and of sugar include oxyethylenated (20 EO) methylglucose sesquistearate, such as the product sold under the name "Glucamate SSE20", by the company Amerchol.

Preferred sugar fatty acid esters for use in the present invention are those selected from the group consisting of sucrose polystearate, sucrose distearate, sucrose dioleate, sucrose pentaoleate, sucrose heptaoleate, sucrose dilinoleate, methyl glucose oleate, methyl glucose isostearate, methyl glucose sesquistearate and mixtures thereof.

The low HLB sugar surfactant is employed in an amount of from 0.2 to 10 %, preferably from 0.2 to 7 %, most preferably from 0.3 to 5 %.

### LOW HLB NONIONIC POLYMERIC SURFACTANT

Non-ionic polymeric surfactants suitable for inclusion into inventive composition are noioninc polymeric surfactants with an HLB below 11, preferably HLB below 9, most preferably below 7 and are selected from the group consisting of block copolymer of type ABA, polysiloxane polyalkyl polyether copolymers and mixtures thereof. The nonionic surfactants described below are suitable for use in the present invention, as long as they are selected also to have HLB below 11.

One type of ABA block copolymer are those having the general formula A-COO-B-OOC-A, in which B is the divalent residue of a water-soluble polyalkylene glycol and A is the residue of an oil-soluble complex monocarboxylic acid i.e. a fatty acid, with a molecular weight of at least 500. These complex monocarboxylic acids may be represented by the general formula: 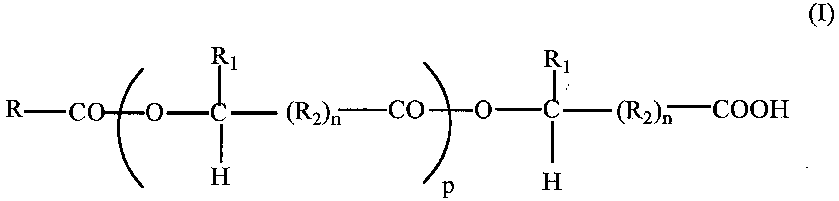 in which
R is hydrogen or a monovalenthydrocarbon or substituted hydrocarbon group;
R₁ is hydrogen or a monovalent C₁ to C₂₄ hydrocarbon group;
R₂ is a divalent C₁ to C₂₄ hydrocarbon group;
n is zero or 1;
p is an integer from zero to 200.

The units between the brackets in formula 1 may be all the same or they may differ in respect of R₁, R₂ and n. The quantity p will not normally have the same unique value for all molecules of the complex acid but will be statistically distributed about an average value lying within the range stated, as is commonplace in polymeric materials. Polymeric component B has a molecular weight of at least 500 and is the divalent residue of a water-soluble polyalkylene glycol having the general formula 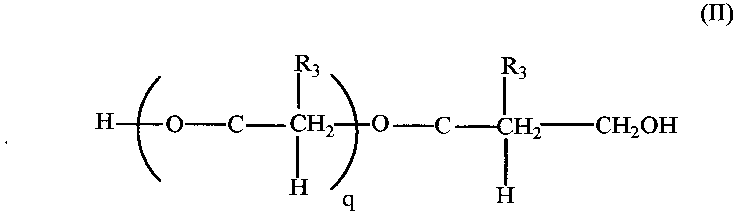 wherein
R₃ is hydrogen or a C₁ to C₃ alkyl group;
q is an integer from 10 up to 500.

The repetitive units in formula II again may all be the same or may differ in R₃, and the quantity q may vary about an average value.

The hydrocarbons R, R₁, and R₂ may be linear or branched.

Preferably in the block copolymers of formula A-COO-B-OOC-A, component B is derived from polyethylene glycol and components A are derived from stearic acid, for example polyhydroxystearic acid, preferably from poly (12-hydroxy-stearic acid).

Thus, R may be a straight chain C₁₇H₃₅-group derived from stearic acid, and the unit containing R₁ and R₂ may be derived from 12-hydroxy-stearic acid.

p, in this case, preferably has a value of at least 2.

Preferably q may have a value between 20 and 60, more preferably above 23. The weight ratio of the combined components A to the component B may vary widely, and typically will lie in the range from 9:1 to 1:9.

Most preferred low HLB nonionic polymeric surfactants used according to the invention are Arlacel P135, a PEG 30 Dipolyhydroxystearate. Another, similar, surfactant for use in the invention is Atlox 4912. Both Arlacel P135 and Atlox 4912 are block copolymers (A-B-A) of polyethylene glycol and polyhydroxystearic acid with a molecular weight of approximately 5000 commercially available from Croda.

Another type of ABA block copolymer are those consisting of propylene oxide (PO) and ethylene oxide (EO) blocks otherwise known as Pluronics. Representative of this class are the polyether surfactants comprising block polymers of two or more different kinds of oxyalkylene repeat units, the ratio of which determining the HLB of the surfactant. The straight chain polyether surfactants are available from BASF Wyandotte Corp., Wyandotte, Mich., under the registered trademark "Pluronic™ (BASF)." For convenience purposes, the straight chain surfactants employed in the aqueous composition disclosed herein will be referred to as Pluronic generally, and with a numerical suffix to identify a particular grade of material.

Pluronic are block copolymers consisting of propylene oxide (PO) and ethylene oxide (EO) blocks --specifically, they are poly(A-oxyethylene-B-oxypropylene-A-oxyethylene)triblock copolymers. Their solubility in water is generally good, but the properties of the individual block copolymers vary substantially. The nomenclature used for the block copolymers, and generally herein, is such that the first two figures, when multiplied by 100, represent the average molecular weight of the PO block, whilst the last figure, when multiplied by 10, represents the ethylene oxide content (% w/w) of the poloxamer. Thus, for Pluronic F127, the average molecular weight of the PO block is 12000 Daltons with 70 % w/w/ethylene oxide content.

Suitable straight chain polyether surfactants having HLB value less than or equal to 11 include for example but are not limited to:
Pluronic L42™ (BASF) having a HLB of 8 and average molecular weight (AMW) of 1630;
Pluronic L63™ (BASF) having a HLB of 11 and average molecular weight (AMW) of 2650;
Pluronic L101™ (BASF) having a HLB of 1 and average molecular weight (AMW) of 3800;
Pluronic P103™ (BASF) having a HLB of 9 and average molecular weight (AMW) of 4950;
Pluronic P123™ (BASF) having a HLB of 8 and average molecular weight (AMW) of 5750;
Pluronic L122™ (BASF) having a HLB of 4 and average molecular weight (AMW) of 5000;
Pluronic L121™ (BASF) having a HLB of 1 and average molecular weight (AMW) of 4400;
Pluronic L92™ (BASF) having a HLB of 6 and average molecular weight (AMW) of 3650;
Pluronic L81™ (BASF) having a HLB of 2 and average molecular weight (AMW) of 2750;
Pluronic L72™ (BASF) having a HLB of 7 and average molecular weight (AMW) of 2750;
Pluronic L62™ (BASF) having a HLB of 7 and average molecular weight (AMW) of 2500;
Pluronic L61™ (BASF) having a HLB of 3 and average molecular weight (AMW) of 2000;
Pluronic L31™ (BASF) having a HLB of 5 and average molecular weight (AMW) of 1100.
Most preferred Pluronic surfactant is Pluronic L61, L62, L72, L81, L92, L101, L121, L122, L123™.

Another type of suitable polymeric surfactant according to the invention are polysiloxane polyalkyl polyether copolymers, i.e., silicone glycol surfactants which are also known as dimethicone copolyols. The amount of silicone glycol surfactant is preferably about 0.5 to 15 % by weight. A more preferable amount of the silicone glycol surfactant is about 0.5-5 % of the composition.

Suitable silicone surfactants are for example high molecular weight polymers of dimethyl polysiloxane with polyoxyethylene and/or polyoxypropylene side chains, having a molecular weight of from 10,000 to 50,000 and having the structure: 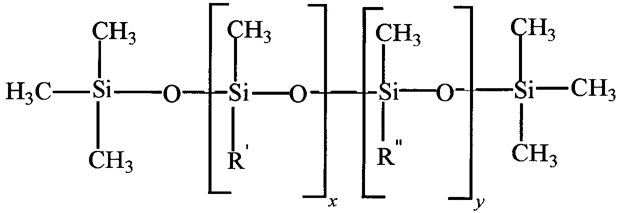 wherein the groups R' are each chosen from --H, C₁₋₁₈ alkyl and
R" is --[CH₂CH₂O]ₐ[CH₂(CH₂)CHO]_{b}H, in which
a has a value of from 9 to 115,
b has a value of from 0 to 50,
x has a value of from 133 to 673,
y has a value of from 25 to 0.25.

Preferably, the polymer is an alkoxylated polydimethyl polymer in which:
a has a value of from 10 to 114,
b has a value of from 0 to 49,
x has a value of from 388 to 402,
y has a value of from 15 to 0.75,
the group R" having a molecular weight of from 1000 to 5000.

A more preferred alkoxylated dimethyl polysiloxane polymer is one in which:
a has the value 14,
b has the value 13,
x has the value 249,
y has the value 1.25.

The most preferred polymeric surfactant for use herein are cetyl dimethicone copolyol (Abil EM90) and PEG30 dipolyhydroxystearate (Arlacel P135) (30 here designates the number of polyethylene glycol repeat units).

The amount of polymeric surfactants is in amount of from 0.1 to 10 %, preferably from 0.2 to 7 %, most preferably from 0.2 to 5 %.

Optionally such polymeric surfactants, such as for example PEG30 dipolyhydroxystearate, can be sold as a mixture with octyldodecanol and octyldodecyl xyloside, sold, for example, under the name Easynov by the company Seppic.

According to the present invention, in order to maintain structural reversibility, the ratio of low HLB nonionic sugar surfactant to low HLB nonionic polymeric surfactant is in the range from about 20:80 to 95:5, preferably from 25:75 to 95:5, most preferably from 25:75 to 90:10.

### Water

The compositions of the present invention are oil-continuous but include generally from 1 to 98 % of water, preferably from 40 to 95 %, most preferably from 52 to 90 %, optimally from 55 to 80 % of water.

### Oil

Oils included as part of this invention may be in the form of silicone oils, esters and hydrocarbons. Amounts of the oils may range anywhere from about 1 to about 95 %, preferably between about 1 and about 50 %, and most preferably from 1-20 % by weight of the composition.

Silicone oils may be divided into the volatile and nonvolatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 6, silicon atoms.

Non-volatile silicone oils useful as an emollient material include polyalkyl siloxanes, polyalkylaryl siloxanes and polyether siloxane copolymers. The essentially nonvolatile polyalkyl siloxanes useful herein include, for example, polydimethyl siloxanes with viscosities of from about 5 x 10⁻⁶ to 0.1 m²/s at 25°C. Among the preferred nonvolatile emollients useful in the present compositions are the polydimethyl siloxanes having viscosities from about 1 x 10⁻⁵ to about 4 x 10⁻⁴ m²/s at 25°C.

Another class of non-volatile silicones are emulsifying and non-emulsifying silicone elastomers. Representative of this category is Dimethicone/Vinyl Dimethicone Crosspolymer available as Dow Corning 9040, General Electric SFE 839, and Shin-Etsu KSG-18. Silicone waxes such as Silwax WS-L (Dimethicone Copolyol Laurate) may also be useful.

Among the ester emollients are:
1) Alkyl esters of saturated fatty acids having 10 to 24 carbon atoms. Examples thereof include behenyl neopentanoate, isopropyl stearate, isopropyl oleate, isononyl isonanonoate, isopropyl myristate and octyl stearate and mixtures thereof.
2) Polyhydric alcohol esters. Ethylene glycol mono and di-fatty acid esters, diethylene glycol mono- and di-fatty acid esters, polyethylene glycol (200-6000) di-fatty acid esters, propylene glycol mono- and di-fatty acid esters, polypropylene glycol 2000 monostearate, ethoxylated propylene glycol monostearate, glyceryl di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, are satisfactory polyhydric alcohol esters. Particularly useful are pentaerythritol, trimethylolpropane and neopentyl glycol esters of C₁-C₃₀ alcohols.
3) Wax esters such as beeswax, spermaceti wax and tribehenin wax.

Hydrocarbons which are suitable oils and include petrolatum, mineral oil, C₁₁-C₁₃ isoparaffins, and especially isohexadecane, available commercially as Permethyl 101 A from Presperse Inc.

Preferred oils that can be used, especially for products intended to be applied to the face, to improve sensory properties and are chosen from the group of oils that do not form stiff gels with 12-HSA; these include polypropylene glycol-14 butyl ether otherwise known as Tegosoft PBE, or PPG15 stearyl ether such as Tegosoft E, other oils such as esters, specifically, isopropyl myristate, isopropyl palmitate, other oils could include castor oils and derivatives thereof.

### Form of the Composition

The compositions of the present invention are non-solid. Essentially, the "non-solidness" of the composition means that the viscosity of the compositions, e.g. as measured using a Brookfield DV-I + viscometer (20RPM, RV6, 30 seconds). The viscosity is in general is in the range of from 1 Pas to 500Pas, preferably from 1 Pas to 200Pas, more preferably from 2Pas to 100Pas, most preferably from 3Pas to 50Pas.

The compositions of the invention are leave-on compositions. The compositions of the present invention are intended to be applied to remain on the skin. These leave-on compositions are to be distinguished from compositions which are applied to the skin and subsequently removed either by washing, rinsing, wiping, or the like either after or during the application of the product. Surfactants typically used for rinse-off compositions have physico-chemical properties giving them the ability to generate foam/lather in-use with ease of rinse; they can consist of mixtures of anionic, cationic, amphoteric, and non-ionic. Surfactants used in leave-on compositions on the other hand are not required to have such properties. Rather, as leave-on compositions are not intended to be rinsed-off they need to be non-irritating and therefore it would be necessary to minimize the total level of surfactant, and particularly the total level of anionic surfactant in leave-on compositions. Therefore, the compositions of the present invention contain, with respect to surfactants, predominantly nonionic surfactants. The anionic surfactants are present in an amount of at most 5 %, preferably from 0.01 to 4 %, more preferably from 0.01 to 3 %, most preferably from 0.01 to 2 % and optimally are substantially absent (less than 1 %, preferably less than 0.1 %, or even less than 0.01 %). Salts of 12HSA are not considered anionic surfactants herein. The total level of surfactant in the inventive compositions is preferably no more than 10 %, more preferably below 8 %, most preferably at most 5 %.

The compositions of the present invention are in the form of emulsions; preferably the compositions are water-in-oil emulsions, including high internal phase emulsions. Another preferred format are acid-soap structured creams, such as a vanishing cream base. Vanishing cream base is one which comprises 5 to 40 % fatty acid and 0.1 to 20 % soap. In such creams, the fatty acid is preferably substantially a mixture of stearic acid and palmitic acid and the soap is preferably the potassium salt of the fatty acid mixture, although other counterions and mixtures thereof can be used. The fatty acid in vanishing cream base is often prepared using hystric acid which is substantially (generally about 90 to 95 %) a mixture of stearic acid and palmitic acid. A typical hystric acid comprises about 52-55 % palmitic acid and 45-48 % stearic acid of the total palmitic-stearic mixture. Thus, inclusion of hystric acid and its soap to prepare the vanishing cream base is within the scope of the present invention. It is particularly preferred that the composition comprises higher than 7 %, preferably higher than 10 %, more preferably higher than 12 % fatty acid.

The compositions of the present invention are stable on storage. The compositions remain stable at 45°C for at least 1 week, more preferably one month, most preferably 3 months, and optimally at least 6 months.

Furthermore, the non-solid leave-on compositions of the present invention are also structurally reversible through temperature cycling between room temperature and 50°C.

### Optional Ingredients

### Additional Surfactants

Suitable additional nonionic detergent active compounds can be broadly described as compounds produced by the condensation of alkylene oxide groups, which are hydrophilic in nature, with an organic hydrophobic compound which may be aliphatic or alkyl aromatic in nature. The length of the hydrophilic or polyoxyalkylene radical which is condensed with any particular hydrophobic group can be readily adjusted to yield an oil-soluble or water-soluble compound. Particular examples include the condensation product of aliphatic alcohols having from 8 to 22 carbon atoms in either straight or branched chain configuration with ethylene oxide, such as a coconut oil ethylene oxide condensate having from 2 to 15 moles of ethylene oxide per mole of coconut alcohol; condensates of alkylphenols whose alkyl group contains from 6 to 12 carbon atoms with 5 to 25 moles of ethylene oxide per mole of alkylphenol; condensates of the reaction product of ethylenediamine and propylene oxide with ethylene oxide, the condensate containing from 40 to 80 % of polyoxyethylene radicals by weight and having a molecular weight of from 5,000 to 11,000; tertiary amine oxides of structure R₃NO, where one group R is an alkyl group of 8 to 18 carbon atoms and the others are each methyl, ethyl or hydroxyethyl groups, for instance dimethyldodecylamine oxide; tertiary phosphine oxides of structure R₃PO, where one group R is an alkyl group of from 10 to 18 carbon atoms, and the others are each alkyl or hydroxyalkyl groups of 1 to 3 carbon atoms, for instance dimethyldodecylphosphine oxide; and dialkyl sulphoxides of structure R₂SO where R₂ is an alkyl group, which can be branched of from 10 to 18 carbon, for instance methyl tetradecyl sulphoxide; another example is the condensation product of fatty acid with amines to form RCONR₁CH₂X where R is C8 to C22 (may be branched or ethoxylated), R₁ is Hydrogen or methyl, ethyl, CH₂CH₂OH; X is CH₂OH, CH₂OR₄ (R₄= ethoxylated i.e CH2CH2OH, CH2CH2OCH2CH2OH).

Suitable/preferred nonionic surfactants include ethoxylated sorbitan fatty acid esters (otherwise known as Tween series from Croda/Uniquema), ethoxylated fatty acids (otherwise known as Brij series from Croda/Uniquema), alternatively other fatty acids can also be optionally added.

### Alkyl Polyglucosides

Alkyl polyglucosides are made from fatty alcohol and sugar. Preferably, the C₈-C₂₂ or C₁₄ -C₂₂ fatty alcohols forming the fatty unit of the alkyl polyglucosides include a saturated or unsaturated branched or linear alkyl chain respectively containing from 8 to 22 or from 14 to 22 carbon atoms. This includes C₈, C₉, C₁₀, C₁₁, C₁₂, C₁₃, C₁₄, C₁₅, C₁₆, C₁₇, C₁₈, C₁₉, C₂₀, C₂₁, and C₂₂ in any subrange the fatty alcohol is a linear fatty alcohol. Preferably, the fatty unit of the alkyl polyglucosides includes any of decyl, cetyl, behenyl, arachidyl, stearyl, palmityl, myristyl, lauryl, capryl or hexadecanyl units and their mixtures, such as cetearyl (C16-C18 mixture).

### Additional fatty acids

In a preferred embodiment, compositions of the present invention can further comprise an acid like a fatty acid, in addition to 12HSA. Illustrative non-limiting examples of fatty acids which may be used include saturated or unsaturated branched or linear alkyl chain respectively containing from 8 to 22 or from 14 to 22 carbon atoms. This includes C₈, C₉, C₁₀, C₁₁, C₁₂, C₁₃, C₁₄, C₁₅, C₁₆, C₁₇, C₁₈, C_{18:1}, C_{18:2}, C_{18:3}, C₁₉, C₂₀, C₂₁, and C₂₂.

### Anionic Surfactant

Anionic materials useful herein are soaps (i.e., alkali metal salts, e.g., sodium or potassium salts or ammonium or triethanolamine salts) of fatty acids, typically having from about 8 to about 24 carbon atoms, preferably from about 10 to about 20 carbon atoms. The fatty acids used in making the soaps can be obtained from natural sources such as, for instance, plant or animal-derived glycerides (e.g., palm oil, coconut oil, soybean oil, castor oil, tallow, lard, etc.). The fatty acids can also be synthetically prepared. Soaps are described in more detail in U.S. Pat. No. 4,557,853.

### Thickening polymer

The thickening polymer is employed in the present invention, in conjunction, with other elements as described herein, to ensure that the inventive compositions are non-solid and are structurally reversible. 12HSA itself does not deliver a thickening functionality in the inventive compositions.

The thickening polymer is selected from the group consisting of biopolymers, synthetic polymers, and mixtures thereof.

The biopolymer can be chosen, for example, from carrageenan, furcellaran, pectin, alginate, agar, agarose, gellan, glucomannan (e.g., Konjac), galactomannan (e.g., locust bean gum, guar), xanthan, modified cellulose, glucan (e.g., starches, curdlan), gelatin, whey protein or mixtures thereof. More preferably, the biopolymer used is Xanthan gum or modified cellulose. The biopolymers suitable for use in this invention are commercially available from suppliers Ashland Aqualon. Additional descriptions of the types of biopolymers that may be used in this invention may be found in Food Gels, Chapter 1, edited by Peter Harris, Elsevier, 1990 and US 6 673 371 and US 5 738 897.

Illustrative synthetic thickeners (or polymeric viscosity builders) which may also be suitably used include alkylated polyvinylpyrrolidones like butylated polyvinyl pyrrolidone sold under the name Ganex® line by ISP Corporation, terephthalate polyesters like polypropylene terephthalate and ammonium acryloyldimethyltaurate/VP Copolymer, both sold under Aristoflex® line by Clariant A.G.; and mono alkyl esters of poly(methyl vinyl/ether maleic acid) sodium salt, like that included in the EZ Sperse® line made available by ISP Corporation, as well as (3-dimethylaminopropyl)-methacrylamide/3-methacryloylamidopropyl)-lauryl-dimethyl-ammonium chloride like that included in the Styleze® line made available by ISP Corporation. Other thickeners suitable for use include those generally classified as acrylic acid/ethyl acrylate copolymers and carboxyvinyl polymers made available by the B.F. Goodrich Company under the Carbopol name. Such thickeners consist essentially of colloidally water-soluble poly-alkenyl polyether cross-linked polymer of acrylic acid crosslinked with a crosslinking agent like polyallyl sucrose or polyallyl pentaerythritol. These thickeners include, for example, Carbopol 934, 940, 950, 951, 980 and 981.

Other examples of suitable synthetic thickeners for use herein include those sold under the name Carbopol Ultrez 10, Carbopol Ultrez 21, Carbopol ETD2020, Carbopol 1342, Carbopol 1382, and Pemulen TR-1 (CTFA designation: Acrylates/10-30 Alkyl Acrylate Cross-polymer). Other examples of suitable thickeners include those made available by Seppic under the names Sepigel 305 and Sepiplus. Still other examples can include polyurethane alkoxylate polymers from BASF otherwise known as polyurethane-39 sold under the tradename Luvigel STAR. If desired, combinations of synthetic thickeners may be also employed.

Preferably, the thickening polymer is selected from a biopolymer or a synthetic thickener, more preferably from a blend of a biopolymer and a synthetic polymer, optimally a mixture of xanthan gum and taurate polymer (e.g. Aristoflex AVC).

The thickening polymer is included in the inventive compositions in an amount of from 0.05 % to 10 %, preferably from 0.1 to 8 %, more preferably from 0.1 to 5 %, optimally from 0.2 to 2 %.

### INORGANIC NEUTRALIZING AGENT

Compositions of the present invention can include an inorganic neutralizing agent, in order to provide better cost. The inorganic neutralizing agent is selected from the group consisting of potassium hydroxide, sodium hydroxide, magnesium chloride, magnesium sulfate, calcium chloride, calcium carbonate, calcium oxide, magnesium oxide, calcium hydroxide, magnesium hydroxide, zinc chloride, zinc oxide, aluminum chloride, aluminum hydroxide, aluminum oxide, and mixtures thereof. Preferably the inorganic neutralizing agent is selected from the group consisting of sodium hydroxide, potassium, hydroxide and zinc oxide. It has been found, as part of the present invention, that an organic neutralizing agent does not provide the desired structural reversibility. Preferably the composition of the invention comprises less than 1, more preferably less than 0.5, even more preferably less than 0.1, most preferably substantially zero % w/w organic neutralizing agent.

As explained above, the compositions of the present invention contain a substantial, preferably pre-dominant amount of 12HSA in acid form. Therefore, the inorganic neutralizing agent, if used, must be used in amount to maintain the preferred pH ranges as discussed above.

### Rheology Modifier

A rheology modifier may be included and is selected from the group consisting of silica such as fumed silica,hydrophilic or hydrophobic silicas and clays such as magnesium aluminum silicate, betonites, hectorite, laponite, and mixtures thereof. A rheology modifier is employed in an amount of from 0.01 to 2 %, preferably from 0.05 to 1 %.

Humectants of the polyhydric alcohol-type can be employed as cosmetically acceptable carriers. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.5 to 50 %, preferably between 1 and 15 % by weight of the composition.

Skin moisturizers, e.g. hyaluronic acid and/or its precursor N-acetyl glucosamine may be included. N-acetyl glucosamine may be found in shark cartilage or shitake mushrooms and are available commercially from Maypro Industries, Inc (New York). Other preferred moisturizing agents include hydroxypropyl tri(C₁-C₃ alkyl)ammonium salts. These salts may be obtained in a variety of synthetic procedures, most particularly by hydrolysis of chlorohydroxypropyl tri(C₁-C₃ alkyl)ammonium salts. A most preferred species is 1,2-dihydroxypropyltrimonium chloride, wherein the C₁-C₃ alkyl is a methyl group. Amounts of the salt may range from about 0.2 to about 30 %, and preferably from about 0.5 to about 20 %, optimally from about 1 % to about 12 % by weight of the topical composition, including all ranges subsumed therein.

Ordinarily the C₁-C₃ alkyl constituent on the quaternized ammonium group will be methyl, ethyl, n-propyl, isopropyl or hydroxyethyl and mixtures thereof. Particularly preferred is a trimethyl ammonium group known through INCI nomenclature as a "trimonium" group. Any anion can be used in the quat salt. The anion may be organic or inorganic with proviso that the material is cosmetically acceptable. Typical inorganic anions are halides, sulfates, phosphates, nitrates and borates. Most preferred are the halides, especially chloride. Organic anionic counter ions include methosulfate, toluoyl sulfate, acetate, citrate, tartrate, lactate, gluconate, and benzenesulfonate.

Still other preferred moisturizing agents which may be used, especially in conjunction with the aforementioned ammonium salts include substituted urea like hydroxymethyl urea, hydroxyethyl urea, hydroxypropyl urea; bis(hydroxymethyl) urea; bis(hydroxyethyl) urea; bis(hydroxypropyl) urea; N,N'-dihydroxymethyl urea; N,N'-di-hydroxyethyl urea; N,N'-di-hydroxypropyl urea; N,N,N'-tri-hydroxyethyl urea; tetra(hydroxymethyl) urea; tetra(hydroxyethyl) urea; tetra(hydroxypropyl urea; N-methyl, N'-hydroxyethyl urea; N-ethyl-N'-hydroxyethyl urea; N-hydroxypropyl-N'-hydroxyethyl urea and N,N'dimethyl-N-hydroxyethyl urea. Where the term hydroypropyl appears, the meaning is generic for either 3-hydroxy-n-propyl, 2-hydroxy-n-propyl, 3-hydroxy-i-propyl or 2-hydroxy-i-propyl radicals. Most preferred is hydroxyethyl urea. The latter is available as a 50 % aqueous liquid from the National Starch & Chemical Division of ICI under the trademark Hydrovance.

Amounts of substituted urea that may be used in the topical composition of this invention range from about 0.01 to about 20 %, and preferably, from about 0.5 to about 15 %, and most preferably, from about 2 to about 10 % based on total weight of the composition and including all ranges subsumed therein.

When ammonium salt and substituted urea are used, in a most especially preferred embodiment at least from about 0.01 to about 25 %, and preferably, from about 0.2 to about 20 %, and most preferably, from about 1 to about 15 % humectant, like glycerine, is used, based on total weight of the topical composition and including all ranges subsumed therein.

### Skin Benefit Ingredients

The inventive composition preferably includes an additional skin lightening compound, to obtain optimum skin lightening performance at an optimum cost. Illustrative substances are placental extract, lactic acid, niacinamide, arbutin, kojic acid, ferulic acid, hydroquinone, resorcinol and derivatives including 4-substituted resorcinols and combinations thereof. More preferably, such additional skin lightening compound is a tyrosinase inhibitor, to complement the melanogenesis inhibition activity of the substituted monoamines, most preferably a compound selected from the group consisting of kojic acid, hydroquinone and 4-substituted resorcinol. Also, dicarboxylic acids represented by the formula HOOC-(CxHy)-COOH where x=4 to 20 and y=6 to 40 such as azelaic acid, sebacic acid, oxalic acid, succinic acid, fumaric acid, octadecenedioic acid or their salts or a mixture thereof, most preferably fumaric acid or salt thereof, especially di-sodium salt. It has been found that combination of 12HSA with fumaric acid or salts thereof are particularly preferred, especially for skin lightening formulations. Amounts of these agents may range from about 0.1 to about 10 %, preferably from about 0.5 to about 2 % by weight of the composition. It is preferred that the skin lightening coactive according to the invention is vitamin B3 or a derivative thereof and is selected from the group consisting of niacinamide, nicotinic acid esters, non-vasodilating esters of nicotinic acid, nicotinyl amino acids, nicotinyl alcohol esters of carboxylic acids, nicotinic acid N-oxide, niacinamide N-oxide and mixtures thereof.

Sunscreen is another preferred ingredient of the inventive compositions. Particularly preferred are such materials as ethylhexyl p-methoxycinnamate (available as Parsol MCX^{®}), Avobenzene (available as Parsol 1789^{®}), octylsalicylate (available as Dermablock OS^{®}), tetraphthalylidene dicamphor sulfonic acid (available as Mexoryl SX®), benzophenone-4 and benzophenone-3 (Oxybenzone). Inorganic sunscreen actives may be employed such as microfine titanium dioxide, zinc oxide, polyethylene and various other polymers. By the term "microfine" is meant particles of average size ranging from about 10 to about 200 nm, preferably from about 20 to about 100 nm. Amounts of the sunscreen agents when present may generally range from 0.1 to 30 %, preferably from 2 to 20 %, optimally from 4 to 10 % by weight of the composition.

More preferred inventive compositions include both the additional skin lightening compound, especially tyrosinase inhibitor, and a sunscreen compound.

Another preferred ingredient of the inventive compositions is a retinoid. As used herein, "retinoid" includes all natural and/or synthetic analogs of Vitamin A or retinol-like compounds which possess the biological activity of Vitamin A in the skin as well as the geometric isomers and stereoisomers of these compounds. The retinoid is preferably retinol, retinol esters (e.g., C₂ -C₂₂ alkyl esters of retinol, including retinyl palmitate, retinyl acetate, retinyl propionate), retinal, and/or retinoic acid (including all-trans retinoic acid and/or 13-cis-retinoic acid), more preferably retinoids other than retinoic acid. These compounds are well known in the art and are commercially available from a number of sources, e.g., Sigma Chemical Company (St. Louis, Mo.), and Boerhinger Mannheim (Indianapolis, Ind.). Other retinoids which are useful herein are described in US 4 677 120, issued June 30, 1987 to Parish et al.; US 4 885 311, issued Dec. 5, 1989 to Parish et al.; US 5 049 584, issued Sep. 17, 1991 to Purcell et al.; US 5 124 356, issued Jun. 23, 1992 to Purcell et al.; and US Reissue 34 075, issued Sep. 22, 1992 to Purcell et al. Other suitable retinoids are tocopheryl-retinoate [tocopherol ester of retinoic acid (trans- or cis-), adapalene {6-[3-(1-adamantyl)-4-methoxyphenyl]-2-naphthoic acid}, and tazarotene (ethyl 6-[2-(4,4-dimethylthiochroman-6-yl)-ethynyl]nicotinate). Preferred retinoids are retinol, retinyl palmitate, retinyl acetate, retinyl propionate, retinal and combinations thereof. The retinoid is preferably substantially pure, more preferably essentially pure. The compositions of this invention may contain a safe and effective amount of the retinoid, such that the resultant composition is safe and effective for regulating keratinous tissue condition, preferably for regulating visible and/or tactile discontinuities in skin, more preferably for regulating signs of skin aging, even more preferably for regulating visible and/or tactile discontinuities in skin texture associated with skin aging. The compositions preferably contain from or about 0.005 % to or about 2 %, more preferably 0.01 % to or about 2 %, retinoid. Retinol is preferably used in an amount of from or about 0.01 % to or about 0.15 %; retinol esters are preferably used in an amount of from or about 0.01 % to or about 2 % (e.g., about 1 %); retinoic acids are preferably used in an amount of from or about 0.01 % to or about 0.25 %; tocopheryl-retinoate, adapalene, and tazarotene are preferably used in an amount of from or about 0.01 % to or about 2 %.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01 % to 2 % by weight of the composition.

Compositions of the present invention may include vitamins. Illustrative vitamins are Vitamin A (retinol), Vitamin B₂, Vitamin B₃ (niacinamide), Vitamin B₆, Vitamin B12, Vitamin C, Vitamin D, Vitamin E, Vitamin K and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. A particularly suitable Vitamin B₆ derivative is Pyridoxine Palmitate. Flavonoids may also be useful, particularly glucosyl hesperidin, rutin, and soy isoflavones (including genistein, daidzein, equol, and their glucosyl derivatives) and mixtures thereof. Total amount of vitamins or flavonoids when present may range from 0.0001 to 10 %, preferably from 0.01 % to 1 %, optimally from 0.1 to 0.5 % by weight of the composition.

Another type of useful substance can be that of an enzyme such as oxidases, proteases, lipases and combinations. Particularly preferred is superoxide dismutase, commercially available as Biocell SOD from the Brooks Company, USA.

Desquamation promoters may be present. Illustrative are the monocarboxylic acids. Monocarboxylic acids may be substituted or unsubstituted with a carbon chain length of up to 16. Particularly preferred carboxylic acids are the alpha-hydroxycarboxylic acids, beta-hydroxycarboxylic or polyhydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic, lactic malic and tartaric acids. A representative salt that is particularly preferred is ammonium lactate. Salicylic acid is representative of the beta-hydroxycarboxylic acids. Amounts of these materials when present may range from about 0.01 to about 15 % by weight of the composition. Other phenolic acids include ferulic acid, salicylic acid, kojic acid and their salts.

A variety of herbal extracts may optionally be included in compositions of this invention. Illustrative are pomegranate, white birch (Betula Alba), green tea, chamomile, licorice and extract combinations thereof. The extracts may either be water soluble or water-insoluble carried in a solvent which respectively is hydrophilic or hydrophobic. Water and ethanol are the preferred extract solvents. Also included may be such materials as resveratrol, alpha-lipoic acid, ellagic acid, kinetin, retinoxytrimethylsilane (available from Clariant Corp. under the Silcare 1M-75 trademark), dehydroepiandrosterone (DHEA) and combinations thereof. Ceramides (including Ceramide 1, Ceramide 3, Ceramide 3B, Ceramide 6 and Ceramide 7) as well as pseudoceramides may also be utilized for many compositions of the present invention but may also be excluded. Amounts of these materials may range from about 0.000001 to about 10 %, preferably from about 0.0001 to about 1 % by weight of the composition.

Colorants, opacifiers and abrasives may also be included in compositions of the present invention. Each of these substances may range from about 0.05 to about 5 %, preferably between 0.1 and 3 % by weight of the composition.

The compositions of the present invention may contain a safe and effective amount of a peptide active selected from pentapeptides, derivatives of pentapeptides, and mixtures thereof. As used herein, "pentapeptides" refers to both the naturally occurring pentapeptides and synthesized pentapeptides. Also useful herein are naturally occurring and commercially available compositions that contain pentapeptides. A preferred commercially available pentapeptide derivative-containing composition is Matrixyl™, which is commercially available from Sederma, France. The pentapeptides and/or pentapeptide derivatives are preferably included in amounts of from about 0.000001 % to about 10 %, more preferably from about 0.000001 % to about 0.1 %, even more preferably from about 0.00001 % to about 0.01 %, by weight of the composition. In embodiments wherein the pentapeptide-containing composition, Matrixyl™, is used, the resulting composition preferably contains from about 0.01 % to about 50 %, more preferably from about 0.05 % to about 20 %, and even more preferably from about 0.1 % to about 10 %, by weight of the resulting composition, of Matrixyl™.

Additional peptides, including but not limited to, di-, tri-, and tetrapeptides and derivatives thereof, and poly amino acid sequences of molecular weight from 200 - 20000. Amino acids may be naturally occurring or synthetic, dextro or levo, straight chain or cyclized and may be included in the compositions of the present invention in amounts that are safe and effective. As used herein, "peptides" refers to both the naturally occurring peptides and synthesized peptides. Also useful herein are naturally occurring and commercially available compositions that contain peptides.

Suitable dipeptides for use herein include Carnosine. Preferred tripeptides and derivatives thereof may be purchased as Biopeptide CL™. and a copper derivative sold commercially as lamin, from Sigma (St.Louis, Mo.).

Further ingredients useful in skin care compositions herein may be selected from any and all: skin conditioning agents, skin feel mildness agents, suspending agents, auxiliary thickening agents, viscosity control agents, dispersants, solubilizing/clarifying agents, stabilizers, opacifiers/pearlescent agents, chelating/sequestering agents, hydrotropes, bactericides/fungicides, antioxidants, pH control agents, buffering agents, colorants and perfumes/fragrances, water, other optional ingredients (auxiliary agents) and the like.

The compositions of the present invention can also be optionally, incorporated into a water insoluble substrate for application to the skin such as in the form of a treated wipe.

### Method of Making Compositions

Compositions within the scope of this invention were prepared in the following manner. Mix all water soluble ingredients including preservatives, glycerine, optionally thickening polymers, and water and heat to a temperature of 70-90°C. In a separate vessel mix all oil soluble ingredients including sugar surfactant and 12HSA to a temperature of 70-90°C. Add the water phase to the oil phase at a temperature of 70-90°C with agitation. Add volatile silicone oil under 67°C. Optionally, add fragrance and phenoxyethanol at 40°C. Cool the mixture to room temperature with mixing.

### Method of Using Compositions

The composition according to the invention is intended primarily as a product for topical application to human skin, especially as an agent for conditioning and smoothening the skin, and preventing or reducing the appearance of wrinkled or aged skin, or age spots, or lightening of the skin.

In use, a small quantity of the composition, for example from 1 to 5 ml, is applied to exposed area of the skin, from a suitable container or applicator and, if necessary, it is then spread over and/or rubbed into the skin using the hand or fingers or a suitable device.

While the above summarizes the present invention, it will become apparent to those skilled in the art that modifications, variations and alterations may be made without deviating from the scope and spirit of the present invention as described and claimed herein. The invention will now be further illustrated in the following non-limiting examples.

### EXAMPLES

Viscosity measurements were made using Brookfield DV-I + Viscometer (20 rpm, RV6, 30 seconds) at room temperature. HLB values of surfactants used in the examples are as follows:
HLB values of surfactants used in the examples are as follows:

| | | |
|---|---|---|
| Surfactant | Tradename | HLB |
| Methyl glucose oleate | Glucate DO | 5 |
| Methyl glucose isostearate | Isolan IS | 5 |
| Methyl glucose sesquistearate | Glucate SS | 6.4 |
| Sucrose polystearate | Sisterna SP10-C | 2 |
| Sucrose distearate | Crodesta F10 | 3 |
| Sorbitan monostearate | Span 60 | 4.7 |
| Peg 7 hydrogenated castor oil | Cremophor WO7 | 4-6 |
| Glyceryl hydroxystearate | GMHS | 3.4 |
| Octyldodecanol, octyldodecyl xyloside and PEG30 dipolyhydroxystearate) - nonionic polymeric surfactant | Easynov | 4.3 |
| Cetyl PEG/PPG-10/1 Dimethicone (cetyl dimethicone copolyol) - nonionic polymeric surfactant | Abil EM90 | 5.0 |
| PEG30 dipolyhydroxystearate - nonionic polymeric surfactant | Arlacel P135 | 5-6 |
| Ethylene oxide / propylene oxide block copolymer | Pluronic L121 | 1 |
| Ethylene oxide / propylene oxide block copolymer | Pluronic L122 | 2 |
| Ethylene oxide / propylene oxide block copolymer | Pluronic P123 | 7 |
| Ethylene oxide / propylene oxide block copolymer | Pluronic L31 | 6-7 |
| Ethylene oxide / propylene oxide block copolymer | Pluronic L62 | 5-6 |

### EXAMPLE 1

Compositions within the scope of the invention were prepared, as detailed in table 1 below.

**TABLE 1**

| Ingredients | Composition (wt %) | | | | | | |
|---|---|---|---|---|---|---|---|
| | 1 | 2 | 3 | 4 | 5 | 6 | 7 |
| Ethyl hexyl cocoate | | | | | 7 | 7 | |
| Isopropyl myristate | 7 | 7 | 7 | 7 | | | 7 |
| Mineral oil 70 | 2.5 | 2.5 | 2.5 | 2.5 | 2.5 | 2.5 | 2.5 |
| Mineral oil 1000 | 2.5 | 2.5 | 2.5 | 2.5 | 2.5 | 2.5 | 2.5 |
| PPG-14 Butyl ether (Tegosoft PBE) | | 5 | 5 | | | | 5 |
| Methyl glucose oleate (low HLB nonionic sugar surfactant) | 0.4 | | | | | | |
| Sucrose distearate (low HLB nonionic sugar surfactant) | | 3 | 1 | 0.4 | 1.5 | 1.75 | 3 |
| Pluronic L-121 (low HLB nonionic polymeric surfactant) | | | | | | | 3 |
| PEG30 dipolyhydroxystearate (low HLB nonionic polymeric surfactant) | | 3 | 2 | | | | |
| Easynov - Octyldodecanol & octyldodecyl xyloside & PEG30 dipolyhydroxystearate (15-25 % PEG30 dipolyhydroxystearate) (low HLB nonionic polymeric surfactant) | 0.9 (0.18) | | | 1.6 (0.32) | 4.5 (0.90) | | 4.5 (0.90) |
| Cetyl PEG/PPG-10/1 dimethicone (cetyl dimethicone copolyol) (low HLB nonionic | | | | | | 5.25 | |
| 12-Hydroxystearic acid | 1 | 3 | 1 | 1 | 1 | 1 | 3 |
| Dimethicone 5 cst oil | 1 | 1 | 1 | | | | |
| Silicone elastomer DC9041 | 5 | 5 | 5 | | | | |
| Cyclopentasiloxane | | | | 6 | 6 | 6 | 6 |
| Clycerine | 7 | 7 | 7 | 7 | 7 | 7 | 7 |
| Dibasic disodium phosphate anhydrous | 0.5 | 0.5 | 0.5 | 0.5 | 0.5 | 0.5 | 0.5 |
| Preservative | 0.7 | 0.7 | 0.7 | 0.7 | 0.7 | 0.7 | 0.7 |
| Fragrance | 0.35 | 0.35 | 0.35 | 0.5 | 0.5 | 0.5 | 0.5 |
| Water | to 100 | to 100 | to 100 | to 100 | to 100 | to 100 | to 100 |
| Viscosity (Pas) | 3.85 | 31.65 | 21.65 | 4.85 | 12.25 | 14.1 | 23.65 |
| Structural Reversibility | Yes | Yes | Yes | Yes | Yes | Yes | Yes |
| Relative ratio low HLB nonionic sugar / low HLB nonionic polymeric (in/out of range) | 69/31 (in range) | 50/50 (in range) | 33/67 (in range) | 56/44 (in range) | 60/40 (in range) | 25/75 (in range) | 77/23 (in range) |
| Low HLB nonionic sugar surfactant | yes | yes | yes | Yes | yes | Yes | yes |
| Low HLB nonionic polymeric surfactant | yes | yes | yes | Yes | yes | yes | yes |

### COMPARATIVE EXAMPLE 2

Compositions outside the scope of the invention were prepared, as detailed in tables 2, 3, 4 and 5:

**TABLE 2 (Comparative)**

| | Composition (wt %) | | | | | |
|---|---|---|---|---|---|---|
| Ingredients | A | B | C | D | E | F |
| Ethyl hexyl cocoate | 7 | 7 | 7 | 7 | 7 | 7 |
| Mineral oil (Paraffinum Liquidum 20) | 2.5 | 2.5 | 2.5 | 2.5 | 2.5 | 2.5 |
| Mineral oil (Paraffinum Liquidum 100) | 2.5 | 2.5 | 2.5 | 2.5 | 2.5 | 2.5 |
| Methyl glucose sesquistearate (low HLB nonionic sugar surfactant) | 2.25 | | | | | |
| Sorbitan monostearate (low HLB nonionic sugar surfactant) | | 2.5 | | | | |
| Sucrose distearate (low HLB nonionic sugar surfactant) | | | 2.4 | | | |
| Sucrose polystearate (low HLB nonionic sugar surfactant) | | | | 2.5 | | |
| Peg-7 hydrogenated castor oil (low HLB non-sugar surfactant) | | | | | | 2.5 |
| Glycerol hydroxystearate (Low HLB non-sugar surfactant) | | | | | 2.5 | |
| Low HLB nonionic polymeric surfactant | 0 | 0 | 0 | 0 | 0 | 0 |
| 12-Hydroxystearic acid | 1 | 1 | 1 | 1 | 1 | 1 |
| Cyclopentasiloxane | 6 | 6 | 6 | 6 | 6 | 6 |
| Glycerine | 7 | 7 | 7 | 7 | 7 | 7 |
| Dibasic disodium phosphate Anhydrous | 0.5 | 0.5 | 0.5 | | 0.5 | 0.5 |
| Sodium chloride | 0 | 0 | 0 | 0.5 | 0 | 0 |
| Preservative | 0.7 | 0.7 | 0.7 | 0.7 | 0.7 | 0.7 |
| Fragrance | 0.35 | 0.35 | 0.35 | 0.35 | 0.35 | 0.35 |
| Water | to 100 | to 100 | to 100 | to 100 | to 100 | to 100 |
| Viscosity (Pas) | -- | -- | -- | -- | -- | -- |
| Structural Reversibility | no | no | no | no | no | no |
| Relative ratio low HLB nonionic sugar / low HLB nonionic polymeric surfactant (in/out of range) | 100/0 (out of range) | 100/0 (out of range) | 100/0 (out of range) | 100/0 (out of range) | 100/0 (out of range) | 100/0 (out of range) |
| Low HLB nonionic sugar | yes | yes | yes | yes | yes | yes |
| Low HLB nonionic polymeric surfactant | no | No | no | no | no | no |

**TABLE 3 (Comparative)**

| Ingredients | Composition (wt %) | | | |
|---|---|---|---|---|
| | G | H | I | J |
| Ethyl hexyl cocoate | 7 | 7 | 7 | 7 |
| Minerla oil 70 | 2.5 | 2.5 | 2.5 | 2.5 |
| Mineral oil 1000 | 2.5 | 2.5 | 2.5 | 2.5 |
| Sucrose distearate (low HLB nonionic sugar surfactant) | 2.5 | 2.4 | 0 | .2 |
| Easynov - Octyldodecanol & octyldodecyl xyloside & PEG30 dipolyhydroxystearate (15-25 % PEG30 dipolyhydroxystearate) (low HLB nonionic polymeric surfactant) | 0.25 (0.05) | 0.1 (0.02) | 2.5 (0.5) | 5(1) |
| 12-Hydroxystearic acid | 1 | 1 | 1 | 1 |
| Cyclopentasiloxane | 6 | 6 | 6 | 6 |
| Glycerine | 7 | 7 | 7 | 7 |
| Dibasic disodium phosphate anhydrous | 0.5 | 0.5 | 0.5 | 0.5 |
| Preservative | 0.7 | 0.7 | 0.7 | 0.7 |
| Fragrance | 0.35 | 0.35 | 0.35 | 0.35 |
| Water | to 100 | to 100 | to 100 | to 100 |
| Viscosity (Pas) | -- | -- | 10.4 | -- |
| Structural Reversibility | No | No | No | No |
| Relative ratio low HLB nonionic sugar / low HLB nonionic polymeric surfactant (in/out of range) | 98/2 (out of range) | 99/1 (out of range) | 0 (out of range) | 17/83 (out of range) |
| Low HLB nonionic sugar | yes | yes | no | yes |
| Low HLB nonionic polymeric surfactant | Yes | yes | yes | yes |

**TABLE 4 (Comparative)**

| Ingredients | Composition (wt %) | | |
|---|---|---|---|
| | K | L | M |
| Isopropyl myristate | 7 | 7 | 7 |
| Minerla oil 70 | 2.5 | 2.5 | 2.5 |
| Mineral oil 1000 | 2.5 | 2.5 | 2.5 |
| Peg-7 hydrogenated castor oil (low HLB nonsugar nonionic surfactant) | 3 | 3 | |
| Methyl glucose sesquistearate (low HLB nonionic sugar surfactant) | 3 | | |
| Sucrose polystearate (low HLB nonionic sugar surfactant) | | 3 | |
| Easynov - Octyldodecanol & octyldodecyl xyloside & PEG30 dipolyhydroxystearate (15-25 % PEG30 dipolyhydroxystearate) (low HLB nonionic polymeric surfactant) | | | 6 (1.2) |
| 12-Hydroxystearic acid | 3 | 3 | 3 |
| Cyclopentasiloxane | 6 | 6 | 6 |
| Glycerine | 7 | 7 | 7 |
| Dibasic disodium phosphate anhydrous | 0.5 | 0.5 | 0.5 |
| Preservative | 0.7 | 0.7 | 0.7 |
| Fragrance | 0.35 | 0.35 | 0.35 |
| Water | to 100 | to 100 | to 100 |
| Viscosity (Pas) | 8.4 | 20.8 | 15.95 |
| Structural Reversibility | no | no | no |
| Relative ratio low HLB nonionic sugar / low HLB nonionic polymeric surfactant | 100/0 | 100/0 | 0/100 |
| Low HLB nonionic sugar | yes | yes | no |
| Low HLB nonionic polymeric surfactant | no | no | yes |

**TABLE 5 (Comparative)**

| Ingredients | Composition (wt %) |
|---|---|
| | N |
| Ethyl hexyl cocoate | 7 |
| Minerla oil 70 | 2.5 |
| Mineral oil 1000 | 2.5 |
| Tegosoft PBE | 3 |
| Cetearyl polyglucoside (nonionic sugar surfactant with HLB = 11) | 2.5 |
| Cetyl PEG /PPG-10/1-dimethicone (cetyl dimethicone copolyol) (low HLB nonionic polymeric surfactant) | 1.5 |
| 12-Hydroxystearic acid | 1 |
| Cyclopentasiloxane | 7 |
| Glycerine | 7 |
| Dibasic disodium phosphate anhydrous | 0.5 |
| Preservative | 0.7 |
| Fragrance | 0.35 |
| Water | to 100 |
| Viscosity (Pas) | -- |
| Structural Reversibility | No |
| Relative ratio low HLB nonionic sugar / low HLB nonionic polymeric surfactant (in/out of range) | 63/37 (in range) |
| Low HLB nonionic sugar | No (nonionic sugar with HLB = 11) |
| Low HLB nonionic polymeric surfactant | Yes |

It can be seen that the compositions outside the scope of the invention did not result in compositions having suitable viscosity and/or structural reversibility.

### EXAMPLE 3

The following additional compositions, as detailed in table 6, within the scope of the invention were prepared.

**TABLE 6**

| Ingredients | Composition (wt %) | | | | | |
|---|---|---|---|---|---|---|
| | 8 | 9 | 10 | 11 | 12 | 13 |
| Ehyl hexyl methoxycinamate | 6 | | | | | |
| Isopropyl myristate | | 7 | 7 | 7 | 7 | 7 |
| Capric caprylic triglyceride | 2.5 | | | | | |
| Cholesterol NF | 0.2 | | | | | |
| Mineral oil 70 | | 2.5 | 2.5 | 2.5 | 2.5 | 2.5 |
| Mineral oil 1000 | | 2.5 | 2.5 | 2.5 | 2.5 | 2.5 |
| Isostearyl alcohol | | 5 | 5 | | | |
| Methyl glucose oleate (low HLB nonionic sugar surfactant) | 1.5 | | | | 1.5 | 0.3 |
| Easynov - Octyldodecanol & octyldodecyl xyloside & PEG30 dipolyhydroxystearate (15-25 % PEG30 dipolyhydroxystearate) (low HLB nonionic polymeric surfactant) | 1 (0.2) | 3 (0.6) | 2 (0.4) | 1.5 (0.3) | 0 | 1 (0.2) |
| Cetyl PEG/PPG-10/1 dimethicone (cetyl dimethicone copolyol) (low HLB nonionic polymeric surfactant) | | | | | 1 | |
| Sucrose distearate (low HLB nonionic sugar surfactant) | | 3 | 1.5 | 1 | | 1.2 |
| 12-Hydroxystearic acid | | 3 | 3 | 1 | 1 | 1 |
| Silicone elastomer DC9041 | 25 | | | | | |
| Cyclopentasiloxane | 0,5 | 6 | 6 | 6 | 6 | 6 |
| Glycerine | | 7 | 7 | | 7 | 7 |
| Dibasic disodium phosphate anhydrous | 0.5 | 0.5 | 0.5 | 0.5 | 0.5 | 0.5 |
| Preservative | 0.2 | 0.5 | 0.5 | 0.5 | 0.2 | 0.2 |
| Fragrance | | 0.35 | 0.35 | 0.35 | | |
| Water | To 100 | to 100 | to 100 | to 100 | to 100 | to 100 |
| Viscosity (Pas) | 36.900 | 18.400 | 16.600 | 3.10 | 5.45 | 4.2 |
| Structural Reversibility | yes | yes | yes | yes | yes | yes |
| Relative ratio low HLB nonionic sugar / low HLB nonionic polymeric surfactant (in/out of range) | 88/12 (in range) | 83/17 (in range) | 78/22 (in range) | 77/23 (in range) | 60/40 (in range) | 88/12 (in range) |
| Low HLB nonionic sugar | yes | yes | yes | yes | yes | yes |
| Low HLB nonionic polymeric surfactant | yes | yes | yes | yes | yes | yes |

While described in terms of the presently preferred embodiments, it is to be understood that such disclosure is not to be interpreted as limiting. Various modifications and alterations will no doubt occur to one skilled in the art after having read the above disclosure.

## Claims

1. Leave-on non-solid oil-continuous skin conditioning composition comprising:
(a) from 0.01 to 15 % by weight of the composition of 12-hydroxystearic acid;
(b) from 0.1 to 30 % by weight of the composition of a nonionic surfactant comprising
(b1) from 0.2 to 10 % by weight of a low HLB nonionic sugar surfactant with an HLB below 9 selected from the group consisting of sugar fatty acid esters, and mixtures thereof,
(b2) from 0.1 to 10 % by weight of a low HLB nonionic polymeric surfactant with an HLB below 11 selected from the group consisting of block copolymer of type ABA, polysiloxane polyalkyl polyether copolymers and mixtures thereof,
(c) wherein the ratio of low HLB nonionic sugar surfactant to low HLB Non-ionic polymeric surfactant is in the range from about 20:80 to 95:5;
(d) wherein the viscosity of the composition is in the range of from about 1 Pas to about 500 Pas at room temperature;
(e) wherein the composition is structurally reversible through temperature cycling between room temperature and 50°C.

2. The composition of claim 1, wherein the sucrose fatty acid ester is selected from the group consisting of sucrose polystearate, sucrose distearate, sucrose diloleate, sucrose pentaoleate, sucrose heptaoleate, sucrose dilinoleateand and mixtures thereof.

3. The composition of claim 1 or claim 2 wherein the low HLB nonionic sugar surfactant is a methyl glucoside ester of a branched or unsaturated fatty acid.

4. The composition of claim 3 wherein methyl glucoside ester is selected from the group methyl glucose oleate and methyl glucose isostearate, methyl glucose hydroxystearate, methyl glucose linoleate, and mixtures thereof.

5. The composition of any one of the preceding claims wherein the low HLB nonionic polymeric surfactant is selected from the group of cetyl dimethicone copolyol, PEG30 dipolyhydroxystearate, ethylene oxide/propylene oxide block copolymer and mixtures thereof.

6. The composition of any one of the preceding claims wherein at least about 40 % of 12-hydroxystearic acid is in acid form.

7. The composition of any one of the preceding claims wherein the composition remains stable at about 45°C for at least 1 week.

8. The composition of any one of the preceding claims further comprising from about 0.05 % to about 10 % of a thickening polymer.

9. The composition of any one of the preceding claims further comprising an inorganic neutralizing agent.

10. The composition of claim 9 wherein the inorganic neutralizing agent is selected from the group consisting of potassium hydroxide, sodium hydroxide, magnesium chloride, magnesium sulfate, calcium chloride, calcium carbonate, calcium oxide, magnesium oxide, calcium hydroxide, magnesium hydroxide, zinc chloride, zinc oxide, aluminum chloride, aluminum hydroxide, aluminum oxide, and mixtures thereof.

## Patentansprüche

1. Auf der Haut verbleibende, nicht-feste Hautkonditionierzusammensetzung mit kontinuierlicher Ölphase, umfassend:
(a) 0,01 bis 15 Gew.-%, bezogen auf die Zusammensetzung, 12-Hydroxystearinsäure;
(b) 0,1 bis 30 Gew.-%, bezogen auf die Zusammensetzung, eines nicht-ionischen Tensids, umfassend
(b1) 0,2 bis 10 Gew.-% eines nicht-ionischen Nieder-HLB-Zuckertensids mit einem HLB-Wert unter 9, ausgewählt aus der Gruppe, die besteht aus Zuckerfettsäureestern und Gemischen davon,
(b2) 0,1 bis 10 Gew.-% eines nicht-ionischen, polymeren Nieder-HLB-Tensids mit einem HLB-Wert unter 11, ausgewählt aus der Gruppe, die besteht aus Blockcopolymeren vom Typ ABA, Polysiloxan-polyalkyl-polyether-Copolymeren und Gemischen davon,
(c) wobei das Verhältnis von nicht-ionischem Nieder-HLB-Zuckertensid zu nicht-ionischem, polymeren Nieder-HLB-Tensid im Bereich von 20:80 bis 95:5 liegt;
(d) wobei die Viskosität der Zusammensetzung im Bereich von etwa 1 Pas bis etwa 500 Pas bei Raumtemperatur liegt;
(e) wobei die Zusammensetzung durch Temperatur-Zyklusbehandlung zwischen Raumtemperatur und 50 °C strukturell reversibel ist.

2. Zusammensetzung nach Anspruch 1, wobei der Saccharosefettsäureester aus der Gruppe ausgewählt ist, die besteht aus Saccharosepolystearat, Saccharosedistearat, Saccharosedioleat, Saccharosepentaoleat, Saccharoseheptaoleat, Saccharosedilinoleat und Gemischen davon.

3. Zusammensetzung nach Anspruch 1 oder Anspruch 2, wobei es sich beim nicht-ionischen Nieder-HLB-Zuckertensid um einen Methylglucosidester einer verzweigten oder ungesättigten Fettsäure handelt.

4. Zusammensetzung nach Anspruch 3, wobei der Methylglucosidester ausgewählt ist aus der Gruppe Methylglucoseoleat und Methylglucoseisostearat, Methylglucosehydroxystearat, Methylglucoselinoleat und Gemischen davon.

5. Zusammensetzung nach einem der vorstehenden Ansprüche, wobei das nicht-ionische, polymere Nieder-HLB-Tensid ausgewählt ist aus der Gruppe Cetyldimethicon-copolyol, PEG30-Dipolyhydroxystearat, Ethylenoxid/Propylenoxid-Blockcopolymeren und Gemischen davon.

6. Zusammensetzung nach einem der vorstehenden Ansprüche, wobei mindestens etwa 40 % der 12-Hydroxystearinsäure in Säureform vorliegen.

7. Zusammensetzung nach einem der vorstehenden Ansprüche, wobei die Zusammensetzung bei etwa 45 °C mindestens eine Woche stabil bleibt.

8. Zusammensetzung nach einem der vorstehenden Ansprüche, ferner umfassend etwa 0,05 bis etwa 10 Gew.-% eines verdickenden Polymers.

9. Zusammensetzung nach einem der vorstehenden Ansprüche, ferner umfassend ein anorganisches Neutralisationsmittel.

10. Zusammensetzung nach Anspruch 9, wobei das anorganische Neutralisationsmittel ausgewählt ist aus der Gruppe, die besteht aus Kaliumhydroxid, Natriumhydroxid, Magnesiumchlorid, Magnesiumsulfat, Calciumchlorid, Calciumcarbonat, Calciumoxid, Magnesiumoxid, Calciumhydroxid, Magnesiumhydroxid, Zinkchlorid, Zinkoxid, Aluminiumchlorid, Aluminiumhydroxid, Aluminiumoxid und Gemischen davon.

## Revendications

1. Composition de traitement de la peau continue en huile non solide sans rinçage comprenant :
(a) de 0,01 à 15 % en poids de la composition d'acide 12-hydroxy-stéarique ;
(b) de 0,1 à 30 % en poids de la composition d'un tensioactif non ionique comprenant
(b1) de 0,2 à 10 % en poids d'un tensioactif de sucre non ionique à faible HLB avec un HLB inférieur à 9 choisi dans le groupe constitué d'esters d'acides gras de sucre, et de mélanges de ceux-ci,
(b2) de 0,1 à 10 % en poids d'un tensioactif polymère non ionique à faible HLB avec un HLB inférieur à 11 choisi dans le groupe constitué d'un copolymère séquencé de type ABA, de copolymères de polysiloxane polyalkyle polyéther et de mélanges de ceux-ci,
(c) dans laquelle le rapport de tensioactif de sucre non ionique à faible HLB au tensioactif polymère non ionique à faible HLB se trouve dans l'intervalle d'environ 20:80 à 95:5 ;
(d) dans laquelle la viscosité de la composition se trouve dans l'intervalle d'environ 1 Pas à environ 500 Pas à température ambiante ;
(e) dans laquelle la composition est structurellement réversible par un cycle de température entre la température ambiante et 50°C.

2. Composition selon la revendication 1, dans laquelle l'ester d'acide gras de saccharose est choisi dans le groupe constitué de polystéarate de saccharose, de distéarate de saccharose, de diloléate de saccharose, de pentaoléate de saccharose, d'heptaoléate de saccharose, de dilinoléate de saccharose et de mélanges de ceux-ci.

3. Composition selon la revendication 1 ou la revendication 2 dans laquelle le tensioactif de sucre non ionique à faible HLB est un ester de méthylglucoside d'un acide gras ramifié ou insaturé.

4. Composition selon la revendication 3, dans laquelle l'ester de méthylglucoside est choisi dans le groupe constitué d'oléate de méthylglucose et d'isostéarate de méthylglucose, d'hydroxystéarate de méthylglucose et de linoléate de méthylglucose, et de mélanges de ceux-ci.

5. Composition selon l'une quelconque des revendications précédentes, dans laquelle le tensioactif polymère non ionique à faible HLB est choisi dans le groupe constitué de copolyol de cétyldiméthicone, de dipolyhydroxystéarate de PEG30, de copolymère séquencé d'oxyde d'éthylène/ oxyde de propylène et de mélanges de ceux-ci.

6. Composition selon l'une quelconque des revendications précédentes, dans laquelle au moins environ 40 % d'acide 12-hydroxy-stéarique sont dans une forme acide.

7. Composition selon l'une quelconque des revendications précédentes, dans laquelle la composition reste stable à environ 45°C pendant au moins 1 semaine.

8. Composition selon l'une quelconque des revendications précédentes comprenant de plus d'environ 0,05 % à environ 10 % d'un polymère épaississant.

9. Composition selon l'une quelconque des revendications précédentes comprenant de plus un agent neutralisant inorganique.

10. Composition selon la revendication 9, dans laquelle l'agent neutralisant inorganique est choisi dans le groupe constitué d'hydroxyde de potassium, d'hydroxyde de sodium, de chlorure de magnésium, de sulfate de magnésium, de chlorure de calcium, de carbonate de calcium, d'oxyde de calcium, d'oxyde de magnésium, d'hydroxyde de calcium, d'hydroxyde de magnésium, de chlorure de zinc, d'oxyde de zinc, de chlorure d'aluminium, d'hydroxyde d'aluminium, d'oxyde d'aluminium, et de mélanges de ceux-ci.
